# EUROPÄISCHE PATENTSCHRIFT

(11) **EP 0 582 863 B1**
(45) Veröffentlichungstag und Bekanntmachung des Hinweises auf die Patenterteilung: **18.09.1996**
(21) Anmeldenummer: 93111623.0
(22) Anmeldetag: 21.07.1993
(51) Int. Cl.: C01C 3/14, C07C 209/62

(54) **Verfahren zur Herstellung von Isocyansäure durch Zersetzen von N,N-disubstituirten Harnstoffen**
Process for the preparation of isocyanic acid by decomposition of N,N-disubstituted ureas
Procédé pour la préparation d'acide isocyanique par décomposition durées N,N-disubstituées

(30) Priorität: 13.08.1992 AT 1630/92
(43) Veröffentlichungstag der Anmeldung: 16.02.1994
(73) Patentinhaber: DSM Chemie Linz GmbH, 4021 Linz (AT)
(72) Erfinder: Hackl, Kurt Alfred, Dipl.-Ing,Dr., A-4020 Linz (AT); Müllner, Martin, Dipl.-Ing. Dr., A-4020 Linz (AT); Schulz, Erich, A-4052 Ansfelden (AT); Stern, Gerhard, Dipl.-Ing.Dr., A-4180 Sonnberg (AT); Falk, Heinz, Prof.Dr., A-4040 Linz (AT)
(74) Vertreter: Kunz, Ekkehard, Dr.

(56) Entgegenhaltungen:
- EP-A- 0 124 704
- EP-A- 0 416 236
- CHEMICAL ABSTRACTS, vol. 81, no. 26, 30. Dezember 1974, Columbus, Ohio, US; abstract no. 172444m, Seite 178 ;
- CHEMICAL ABSTRACTS, vol. 82, no. 26, 30. Juni 1975, Columbus, Ohio, US; abstract no. 173135u, Seite 129 ;
- CHEMICAL ABSTRACTS, vol. 81, no. 26, 30. Dezember 1974, Columbus, Ohio, US; abstract no. 172444m, Seite 178 ; & JP-A-7407000
- CHEMICAL ABSTRACTS, vol. 82, no. 26, 30. Juni 1975, Columbus, Ohio, US; abstract no. 173135u, Seite 129 ; & JP-A-7445078

## Beschreibung

Die Erfindung betrifft ein Verfahren zur Herstellung von Isocyansäure durch Zersetzen von N,N-disubstituierten Harnstoffen.

Isocyansäure stellt auf Grund ihrer hohen Reaktivität einen wertvollen C-1 Baustein für die Synthese einer Vielzahl von Verbindungen dar.
Ihre Herstellung kann nach EP-A-0 124 704 durch Erhitzen von geschmolzenem Harnstoff in einer Wirbelschicht erfolgen, wobei ein Isocyansäure-Ammoniak-Gemisch erhalten wird. Die Isolierung der Isocyansäure aus diesem Gemisch bereitet jedoch Schwierigkeiten, da sich beim Abkühlen des Ammoniak-Isocyansäuregemisches Ammoniumisocyanat bildet, das sehr leicht wieder zu Harnstoff isomerisiert.

Aus Chemical Abstracts Vol. 81 (1974) 172444m und Vol. 82 (1975) 173135u ist die thermische Zersetzung von Harnstoff unter Abtrennung von Ammoniak bekannt, wobei sich jedoch ein Feststoff, nämlich Cyanursäure, bildet, die bei 330 bis 600 °C zu Isocyansäure zersetzt werden muß. Die Zersetzung verläuft nur langsam und unvollständig. In EP-A-0416236 werden zwar verbesserte Abtrennungsverfahren für Ammoniak aus Isocyansäure-Ammoniak Gemischen durch Zugabe von tertiären Aminen oder Ethern beschrieben, Aufgabe der vorliegenden Erfindung war es aber, ein neues, einfach durchführbares Verfahren zur Herstellung von Isocyansäure zu finden, bei dem Isocyansäure ohne nachträglichen Trennungsschritt in hoher Ausbeute und Reinheit erhalten wird.

Gegenstand der Erfindung ist demnach ein Verfahren zur Herstellung von Isocyansäure, das dadurch gekennzeichnet ist, daß N,N-disubstituierte Harnstoffe bei erhöhter Temperatur in ein schwererflüchtiges sekundäres Amin und in Isocyansäure, die über Kopf abgezogen wird, zersetzt werden.

Als Ausgangsverbindungen zur Herstellung von Isocyansäure nach dem erfindungsgemäßen Verfahren eignen sich prinzipiell alle N,N-disubstituierten Harnstoffe, die bei thermischer Belastung in Isocyansäure und in ein schwererflüchtiges sekundäres Amin zerfallen.

Bevorzugt werden N,N-disubstituierte Harnstoffe der Formel I 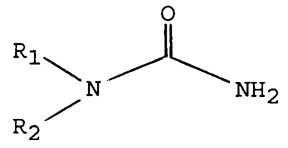
in der R₁ und R₂ gleich oder verschieden sind und einen geradkettigen, verzweigten oder cyclischen, unsubstituierten oder mit (C₁ - C₆) Alkoxy oder mit Phenyl substituierten (C₁ - C₂₄) Alkylrest oder einen unsubstituierten oder mit (C₁ - C₆) Alkyl, (C₁ - C₆) Alkoxy oder mit Hydroxy, oder Halogen, wie Chlor oder Brom oder mit Nitro oder Amin substituierten Phenylrest bedeuten.
Alkylreste sind zum Beispiel Methyl, Ethyl, Propyl, Isopropyl, Butyl, sek.-Butyl, tert.-Butyl, Hexyl, Octyl, Decyl, Dodecyl, Hexadecyl, Octadecyl, Cyclohexyl oder Cycloheptylreste. (C₁ - C₆)Alkoxyradikale sind zum Beispiel Methoxy, Ethoxy, Propoxy, Butoxy, Hexyloxy. Substituierte Phenylreste sind zum Beispiel Tolyl, Aminophenyl, Chlorphenylreste.
Besonders bevorzugte Ausgangsverbindungen sind zum Beispiel N,N-Dibutylharnstoff, N,N-Dihexylharnstoff, N,N-Dioctylharnstoff, N,N-Didodecylharnstoff, N,N-Dihexadecylharnstoff, N,N-Dioctadecylharnstoff, N,N-Dicyclohexylharnstoff und N,N-Dibenzylharnstoff. Die substituierten Harnstoffe können zum Beispiel über N-Alkylierung von Harnstoff, wie in EP 0 471 983 beschrieben, hergestellt werden.

Die substituierten Harnstoffe können, ohne Verwendung eines Verdünnungsmittels_{,} in flüssiger Form oder als Schmelze eingesetzt werden.
Die Zersetzung kann aber auch in einem unter Reaktionsbedingungen inerten Verdünnungsmittel erfolgen. Als solche kommen aliphatische oder aromatische Kohlenwasserstoffe, wie etwa Dodecan, Hexadecan, Octadecan, Toluol und Xylole oder Ether wie z.B. Diethylenglycoldibutylether oder Paraffine oder Mischungen derselben in Betracht. Bevorzugt werden Hexadecan und Diethylenglycoldibutylether eingesetzt.
Es kann aber auch das Amin, das bei der Zersetzung des Harnstoffes entsteht, als Verdünnungsmittel verwendet werden.

Es ist weiters möglich, dem Reaktionsgemisch zusätzlich ein Lösungsmittel für Isocyansäure zuzugeben, welches die Polymerisation der Isocyansäure verhindert oder durch Komplexbildung die Isocyansäure stabilisiert und dann nach beendeter Reaktion in der Kühlfalle gemeinsam mit der Isocyansäure kondensiert, wodurch eine klare, gut handhabbare Lösung reiner Isocyansäure erhalten wird. Lösungsmittel für die Isocyansäure sind beispielsweise aliphatische oder aromatische Kohlenwasserstoffe, die gegebenenfalls auch halogeniert sein können, wie etwa Chloroform, Methylenchlorid, Toluol und Xylole und Ether, wie etwa Tetrahydrofuran, Dioxan, Diisopropylether, Diethylether, tert.Butylmethylether, Diethoxyethan, Dimethoxyethan, Diethylenglycoldialkylether oder Triethylenglycolalkylether mit der Einschränkung, daß der Siedepunkt des Lösungsmittels nicht höher als die angewandte Reaktionstemperatur liegt. Bevorzugt wird jedoch Toluol oder ein Ether wie Diethylenglycoldibutylether, Diethylether, Diethoxyethan oder Dimethoxyethan zugesetzt. Es wird dabei soviel Lösungsmittel zugegeben, sodaß in der Kühlfalle eine maximal 10%ige Lösung an Isocyansäure erhalten wird.

Die Reaktionstemperatur liegt je nach eingesetztem Harnstoff etwa zwischen 90 bis 400 °C, bevorzugt etwa zwischen 150 bis 300 °C und besonders bevorzugt zwischen 180 bis 260 °C.
Die Isolierung der entstehenden Isocyansäure kann auf mehrere Arten erfolgen.
So genügt es zum Beispiel, die Isocyansäure oder das Isocyansäure-Lösungsmittelgemisch abzudestillieren und in einer mit flüssigem Stickstoff gekühlten Kühlfalle zu kondensieren oder in einem der vorgenannten, geeigneten Lösungsmittel, dessen Siedepunkt jedoch auch über der Reaktionstemperatur liegen kann, zu absorbieren.
Zur Verbesserung der Auftrennung von sekundärem Amin und Isocyansäure kann auch eine Fraktionierkolonne verwendet werden, wodurch eventuell mitgeschlepptes Amin durch das kondensierende Verdünnungsmittel wieder in das Reaktionsgemisch zurückgewaschen wird, oder die Isocyansäure wird mittels eines Inertgasstromes, etwa mittels eines Stickstoffstromes oder eines CO₂-Stromes aus dem Reaktionsgemisch entfernt.

Die eben angeführten Maßnahmen zur Isolierung der Isocyansäure können jede für sich, aber auch kombiniert angewandt werden.
Die oben angeführten Verfahrensvariationen können sowohl diskontinuierlich als auch kontinuierlich, z.B. in einem Dünnschichtverdampfer oder in einer Füllkörperkolonne, durchgeführt werden.

Isocyansäure wird dabei mit hohen Ausbeuten und hoher Reinheit erhalten. Die so gewonnene Isocyansäure kann dann rein oder als Lösung entweder bei Temperaturen von -80 °C bis -20 °C einige Wochen gelagert werden oder bei Absorption in einem Lösungsmittel durch Zugabe des entsprechenden Reaktionspartners sofort weiterverarbeitet werden.
Das sekundäre Amin, das als zweites Produkt bei der Zersetzung entsteht, kann zum Beispiel durch Destillation unter reduziertem Druck gereinigt werden und als Ausgangsverbindung für verschiedene Reaktionen eingesetzt werden, oder der Sumpf, der neben dem sekundären Amin auch geringe Mengen an unumgesetztem substituierten Harnstoff und gegebenenfalls ein Verdünnungsmittel enthält, kann direkt ohne weitere Aufarbeitung wieder zur Herstellung neuer substituierter Harnstoffe, etwa nach EP 410.168, verwendet werden.

### Beispiel 1

### Diskontinuierliche Herstellung von Isocyansäure

In einer geeigneten Apparatur, bestehend aus einem Kolben mit Einleitungsmöglichkeiten für das Inertgas und das zusätzliche Lösungsmittel für die Isocyansäure, einem Thermometer, einer aufgesetzten beheizbaren 20 cm langen Vigreuxkolonne und einem absteigenden Kühler, wurde eine 2 %ige Lösung (1 g/49 g Verdünnungsmittel) von Dibenzylharnstoff in Hexadecan vorgelegt und unter Einleiten eines Stickstoffstromes (17 l/h) auf 200°C aufgeheizt. Zusätzlich wurde Diethylether (80 ml/h) in den Sumpf zudosiert.
Die entstehende Isocyansäure und Diethylether wurde in einer mit Eiswasser gekühlten und mit Diethylether beschickten Gaswaschflasche absorbiert.
Zur Ausbeutebestimmung wurde die organische Phase aus der Gaswaschflasche mit Wasser extrahiert und der Gehalt an Isocyansäure mit AgNO₃ potentiometrisch titriert oder die organische Phase wurde mit wäßriger NaOH extrahiert und der Überschuß an NaOH mit HCl rücktitriert.
Ausbeute: 86 %
Analog zu Beispiel 1 wurden folgende Beispiele durchgeführt:

**Tabelle 1**

| **Harnstoff** | **Verdünnungs mittel** | **Konz.d. Lösung (Gew.%)** | **T(°C)** | **N**_{**2**}**-Fluß** | **Isocyansäurelösungsmittel** | **Ausbeut (%)** |
|---|---|---|---|---|---|---|
| 2.Dibenzylharnstoff | Hexadecan | 5 | 250 | 7 l/h | Diethylether 40 ml/h | 57 |
| 3.Dibenzylharnstoff | Hexadecan | 10 | 200 | 17 l/h | Diethylether 80 ml/h | 50 |
| 4.Dibenzylharnstoff | Hexadecan | 5 | 200 | 17 l/h | Diethylether 40 ml/h | 69 |
| 5.Dibenzylharnstoff | Hexadecan | 5 | 200 | 34 l/h | - | 69 |
| 6.Dibenzylharnstoff | Hexadecan | 5 | 200 | 17 l/h | Diethoxyethan 80 ml/h | 80 |
| 7.Dioctylharnstoff | Hexadecan | 5 | 200 | 17 l/h | Diethylether 80 ml/h | 65 |

### Beispiel 8

### Kontinuierliche Herstellung in einem Dünnschichtverdampfer

Während 1 Stunde wurde eine 5 Gew.%ige Lösung von Dioctylharnstoff in Hexadecan in einen Dünnschichtverdampfer dosiert, der auf einer Temperatur von 230 °C gehalten wurden. Der Inertgasstrom im Dünnschichtverdampfer betrug 17 l/h N₂. Die entstehende Isocyansäure wurde in einer mit Wasser (15 °C) gekühlten mit 50 ml Toluol beschickten Vorlage absorbiert.
Zur Ausbeutebestimmung wurde die Toluollösung mit Natronlauge extrahiert und der Gehalt an Isocyansäure durch Rücktitration mit Salzsäure bestimmt.
Ausbeute: 50 %

### Beispiel 9

### Kontinuierliche Herstellung in einer Füllkörperkolonne

Eine mit Raschigringen beschickte Füllkörperkolonne mit absteigendem Kühler wurde auf 250 °C beheizt und am Kopf mit einer 5 Gew%igen Lösung von Dioctylharnstoff in Hexadecan beaufschlagt (10 ml/h). In den Sumpf wurden 17 l/h N₂ eingeleitet. Das Strippergas und die entstehende Isocyansäure wurden im Kühler auf Raumtemperatur gekühlt und in einer mit Diethylether befüllten und mit Eiswasser gekühlten Gaswaschflasche absorbiert.
Die Ausbeutebestimmung erfolgte analog Beispiel 8.
Ausbeute: 86 %

### Beispiel 10

Wurde analog Beispiel 9 durchgeführt.
5 Gew.%ige Lösung ans Dioctylharnstoff in Diethylenglycoldibutylether, 10 ml/h Belastung, 5 l/h N₂, 200 °C Reaktionstemperatur.
Ausbeute: 48 %

### Beispiel 11

Wurde analog Beispiel 9 durchgeführt.
5 Gew%ige Lösung and Dibenzylharnstoff in Diethylenglykoldibutylether, 20 ml/h Belastung, 80 ml/h Diethylether als Isocyansäurelösungsmittel, 17 l/h N₂, 200 °C Reaktionstemperatur.
Ausbeute: 75 %

### Beispiel 12

Wurde analog zu Beispiel 9 durchgeführt.
5 Gew.%ige Lösung an Dioctylharnstoff in Diethylenglykoldibutylether, 10 ml/h Belastung, 17 l/h N₂, 80 ml/h Diethylglykoldibutylether als Isocansäurelösungsmittel, 240 °C Reaktionstemperatur.
Ausbeute: 80 %

### Beispiel 13

Wurde analog Beispiel 9 durchgeführt.
10 Gew.%ige Lösung an Dioctylharnstoff in Diethylenglykoldibutylether, 20 ml/h Belastung, 80 ml/h Diethylenglykoldibutylether als Isocansäurelösungsmittel, 17 l/h N₂, 240 °C Reaktionstemperatur.
Ausbeute: 68 %

## Patentansprüche

1. Verfahren zur Herstellung von Isocyansäure, dadurch gekennzeichnet, daß N,N-disubstituierte Harnstoffe bei erhöhter Temperatur in ein schwererflüchtiges, sekundäres Amin und in Isocyansäure, die über Kopf abgezogen wird, zersetzt werden.

2. Verfahren nach Anspruch 1, dadurch gekennzeichnet, daß N,N-disubstituierte Harnstoffe der Formel I 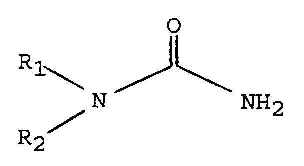 in der R₁ und R₂ gleich oder verschieden sind und einen geradkettigen, verzweigten oder cyclischen, unsubstituierten oder mit (C₁ - C₆) Alkoxy oder Phenyl substituierten (C₁ - C₂₄) Alkylrest oder einen unsubstituierten oder mit (C₁ - C₆) Alkyl, (C₁ - C₆) Alkoxy, Hydroxy, Halogen, Nitro oder Amin substituierten Phenylrest bedeuten, eingesetzt werden.

3. Verfahren nach Anspruch 2, dadurch gekennzeichnet, daß N,N-disubstituierte Harnstoffe der Formel I in der R₁ und R₂ einen geradkettigen, verzweigten oder cyclischen (C₄ - C₂₀) Alkylrest oder einen Benzylrest bedeuten, eingesetzt werden.

4. Verfahren nach Anspruch 1, dadurch gekennzeichnet, daß die Harnstoffzersetzung ohne Verdünnungsmittel oder in einem unter Reaktionsbedingungen inerten Verdünnungsmittel, gegebenenfalls in Kombination mit einem Lösungsmittel für Isocyansäure und/oder einem Inertgasstrom durchgeführt wird.

5. Verfahren nach Anspruch 1, dadurch gekennzeichnet, daß die Reaktionstemperatur etwa 150 bis 300 °C, besonders bevorzugt 180 bis 260 °C, beträgt.

## Claims

1. Process for the preparation of isocyanic acid, characterized in that N,N-disubstituted ureas are decomposed at elevated temperature to a secondary amine of lower volatility and to isocyanic acid, which is drawn off at the top.

2. Process according to Claim 1, characterized in that the N,N-disubstituted ureas used are those of formula I: 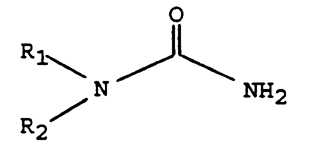 in which R₁ and R₂ are identical or different and are a linear, branched or cyclic (C₁-C₂₄)-alkyl radical which is unsubstituted or substituted by (C₁-C₆)-alkoxy or phenyl, or a phenyl radical which is unsubstituted or substituted by (C₁-C₆)-alkyl, (C₁-C₆)-alkoxy, hydroxyl, halogen, nitro or amino.

3. Process according to Claim 2, characterized in that the N,N-disubstituted ureas used are those of formula I in which R₁ and R₂ are a linear, branched or cyclic (C₄-C₂₀)-alkyl radical or a benzyl radical.

4. Process according to Claim 1, characterized in that the decomposition of the urea is carried out without a diluent or in a diluent which is inert under the reaction conditions, optionally in combination with a solvent for the isocyanic acid and/or with a stream of inert gas.

5. Process according to Claim 1, characterized in that the reaction temperature is about 150 to 300°C, particularly preferably 180 to 260°C.

## Revendications

1. Procédé de préparation d'acide isocyanique, caractérisé en ce que des urées N,N-disubstituées sont décomposées, à une température élevée, en une amine secondaire peu volatile et en acide isocyanique qui est soutiré par la tête de colonne.

2. Procédé selon la revendication 1, caractérisé en ce qu'on utilise des urées N,N-disubstituées répondant à la formule I 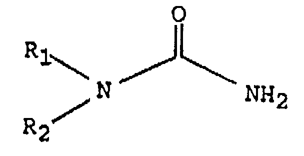 dans laquelle R₁ et R₂ sont identiques ou différents et signifient un reste alkyle en C₁-C₂₄ linéaire, ramifié ou cyclique, non substitué ou substitué par un reste alcoxy en C₁-C₆ ou substitué par un reste phényle, ou un reste phényle non substitué ou substitué par un reste alkyle en C₁-C₆, par un reste alcoxy en C₁-C₆ ou par un reste hydroxy, un halogène, un groupe nitro ou une amine.

3. Procédé selon la revendication 2, caractérisé en ce qu'on utilise des urées N,N-disubstituées de formule I où R₁ et R₂ signifient un reste alkyle en C₄-C₂₀ linéaire, ramifié ou cyclique, ou un reste benzyle.

4. Procédé selon la revendication 1, caractérisé en ce qu'on effectue la décomposition des urées sans diluant ou alors dans un diluant inerte dans les conditions de réaction, éventuellement combiné avec un solvant pour l'acide isocyanique et/ou un flux de gaz inerte.

5. Procédé selon la revendication 1, caractérisé en ce que la température de réaction est d'environ 150 à 300°C, d'une manière particulièrement préférée de 180 à 260°C.
